# EUROPÄISCHE PATENTSCHRIFT

(11) **EP 0 763 600 B1**
(45) Veröffentlichungstag und Bekanntmachung des Hinweises auf die Patenterteilung: **25.06.2003**
(21) Anmeldenummer: 96114583.6
(22) Anmeldetag: 12.09.1996
(51) Int. Cl.: C12P 7/38

(54) **Verfahren zur Präparation und Fermentation von 13-substituierten 3-Methoxy-8, 14-seco-1, 3, 5 (10), 9(11)-gonatetraen-14, 17-dionen**
Process for the preparation and fermentation of 13 substituted 3-methoxy-8, 14-seco-1, 3, 5(10), 9(11)-gonatetraene-14, 17-diones
Procédé de préparation et de fermentation de 3-méthoxy-8, 14-séco-1, 3 ,5(10), 9(11)-gonatétraène-14, 17-diones substitués en 13

(30) Priorität: 14.09.1995 DE 19534066
(43) Veröffentlichungstag der Anmeldung: 19.03.1997
(73) Patentinhaber: Jenapharm GmbH & Co. KG, 07745 Jena (DE)
(72) Erfinder: Junne, Wolf, 07743 Jena (DE); Eckert, Hermann, 07743 Jena (DE); Schäfer, Heinz-Peter, 99423 Weimar (DE); Oehler, Lothar, 07749 Jena (DE)

(56) Entgegenhaltungen:
- WO-A-93/20222
- FR-A- 1 565 986
- DATABASE WPI Section Ch, Week 9247 Derwent Publications Ltd., London, GB; Class A25, AN 92-382815 XP002092650 & DD 300 584 A (CHEM WERKE BUNA GMBH) , 25. Juni 1992
- DATABASE WPI Section Ch, Week 8130 Derwent Publications Ltd., London, GB; Class B01, AN 81-54589D XP002092651 5 & SU 778 743 A (AKRIKHIN CHEM-PHARM WKS)
- CHEMICAL ABSTRACTS, vol. 111, no. 21, 20. November 1989 Columbus, Ohio, US; abstract no. 192994, MEHDI, I. ET AL: "Microbial transformation of 13-ethyl-3-methoxy-8,14-seco-gona- 1,3,5(10),9(11)-tetraene-14,17-dione to its 17-.beta. hydroxy derivative by Pichia farinosa in pilot plant fermentors" XP002092649 & INDIAN J. EXP. BIOL. (1989), 27(8), 742-3 CODEN: IJEBA6;ISSN: 0019-5189,1989,

## Beschreibung

Es wird ein neues Verfahren zur Präparation und Fermentation von 13-substituierten 3-Methoxy-8,14-seco-1,3,5(10),9(11)-gona-tetraen-14,17-dionen in wäßrigen Fermentationsmedien durch Suspendieren des Steroids im Wasser in Gegenwart eines Emulgators. Dabei werden 1 Teil der Steroidverbindung 2 bis 9 Teilen eines Wasser-Emulgator-Tensid-Gemisches, welches auf 80 °C bis 95 °C erhitzt wurde, unter Rühren zugesetzt und geschmolzen; die Temperatur wird unter Rühren eine kurze Zeit beibehalten, danach wird ein Kühlprozeß eingeleitet, bei dem durch Dispergieren des Gemisches eine feine Tropfenverteilung erzeugt wird. Nachfolgend wird die wäßrige Steroidsuspension in das Fermentationsmedium eingetragen.

Das erfindungsgemäße Verfahren eignet sich zur Synthese in der Steroid- und in der Naturstoffchemie.

Eine Grundvoraussetzung zur mikrobiologischen Umsetzung von wasserunlöslichen organischen Verbindungen ist eine entsprechende Verteilung dieser organischen Verbindungen im mikrobiologischen System.

Aus der Fach- und Patentliteratur sind verschiedenartige Verfahrensweisen der mechanischen Zerkleinerung in Mühlen bekannt (WO 93/20222).

Die Nachteile dieser Lösungen bestehen darin,
- daß die Methodik jedoch nur für Substanzen aus harten Kristallen anwendbar ist, da sonst die Gefahr der Verklebungen der Mühlen gegeben ist,
- daß sehr viele Fremdkeime mit der zerkleinerten Substanz dem Fermentationsmedium zugeführt werden, welches letztlich zu einer erhöhten Kontaminationsrate führt.

Sehr verbreitet sind Verfahrensweisen, in denen die umzusetzende Substanz dem Fermetationsmedium in gelöster Form zugeführt werden.

Die US-PS 3 481 974 beschreibt beispielsweise ein Verfahren, in der die mikrobiologisch umzusetzende Substanz in erheblichen Mengen eines organischen Lösungsmittels (5 Teile Steroidverbindung in 1600 Teilen Methanol) gelöst werden und dann nach Zusatz zu dem wäßrigen Fermetationsmedium ausfällt.

Nachteilig an dieser Lösung ist, daß
- auch hier die Gefahr von Substanzverklumpungen unterschiedlicher Größe besteht, die die mikrobiologische Umsetzung behindern,
- damit verbunden eine gleichmäßige Verteilung der Substanz nicht gegeben ist,
- die verwendeten organischen Lösungsmittel zumindest am Anfang der Umsetzung meist toxisch auf den Mikroorganismus wirken, was zu weiteren Nachteilen führt,
- da die verwendeten organischen Lösungsmittel während der Umsetzung zum größten Teil mit der Fermentationsabluft ausgetrieben werden, diese Verfahrensweise eine erhöhte Umweltbelastungdarstellt bzw. einen erhöhten Aufwand für die Abluftreinigung erfordert.

Weiterhin beschreibt die DD-PS 232 166 ein Verfahren zur Mikronisierung von Steroiden, bei dem das Steroid in einem organischen Lösungsmittel gelöst, mit einem Tensid versetzt und anschließend sprühgetrocknet wird. Dabei wird ein sehr feines Pulver erhalten, das dann dem Fermentationsmedium zugesetzt wird.

Nachteilig erweist sich hierbei,
- daß auch hier die Möglichkeit der Klumpenbildung gegeben ist
- und daß die Kontamination des Fermentationsmediums bei Eintrag des Pulvers nicht auszuschließen ist.

Eine Keimminderung bzw. Sterilität des dem Fermentationsmedium zugegebenen Steroids kann erreicht werden, wenn das Steroid vor dem Zusatz zum Fermentationsmedium in einer wäßrigen Phase geschmolzen wird.

Die DD-PS 343 568 beschreibt ein Verfahren, bei welchem das Steroid als geschmolzene Substanz in heißem Zustand dem Fermentationsmedium zugesetzt wird. Dabei erstarrt das Steroid.

Der Nachteil besteht hierbei darin,
- daß auch hier die Steroidklumpenbildung nicht verneint werden kann
- daß bei Anwendung dieser Verfahrensweise für niedriger schmelzende Substanzen oder Substanzen, die nicht sofort zu harten Kristallen erstarren, Verklebungen an den inneren Oberflächen des Fermenters gegeben sind
- und somit eine sichere Präparation und Fermentation des Steroids nicht vollständig realisiert werden kann und Ausbeuteverluste zu verzeichnen sind.

Ferner beschreibt die DD-PS 300 584 A ein Verfahren, bei welchem die Emulsion heiß der vorbereiteten Mikrokultur zugesetzt wird. Es wird eine Neigung zur Schaumbildung und Klumpenbildung vorhergesagt.

In FR-A-1 565 986, in SU 778 743 B und in CHEMICAL ABSTRACTS, vol. 111, no. 192994 gwerden Verfahren aufgezeigt, in welchen keine Vorbereitung der Steroide vorgenommen werden.

Der Erfindung liegt die Aufgabe zugrunde, ein Verfahren zu entwickeln, welches die Präparation und Fermentation von 13-substituierten 3-Methoxy-8,14-seco-1,3,5(10),9(11)-gonatetraen-14,17-dionen mit einer gezielten nahezu vollständigen Umsetzung des Steroids im Fermentationsmedium realisiert.

Eine weitere Aufgabe der Erfindung ist es, ein Verfahren zu erhalten, welches gewährleistet, das Steroid als wäßrige Suspension in einem definierten Kornspektrum dem Fermentationsmedium zuzusetzen.

Die Aufgabe wird erfindungsgemäß durch das Verfahren zur Präparation und Fermentation von 13-substituierten 3-Methoxy-8,14-seco-1,3,5(10),9(11)-gonatetraen-14,17-dionen in wäßrigen Frementationsmedien durch Suspendieren des Steroids im Wasser in Gegenwart von Tensiden gemäß Anspruch 1 gelöst, wobei man 1 Teil Steroidverbindung 2 bis 9 Teilen eines auf 80 bis 95 °C erhitzten Wasser-Emulgator-Antischaummittels mit Prohalyt G 20-40®, Tween® als Emulgator und Polypropylenglycol, Polypropylenglycol/Maiskeimöl als Antischaummittel im Verhältnis 2,5 : 1 bis 1 : 2 und einer Menge von 1 bis 5 % bezogen auf Wasser, wobei weiterhin die Menge an Emulgator bestimmt wird durch den Gehalt der Steroidverbindung, in herkömmlicher Weise unter Rühren zusetzt und schmilzt, wobei man den Schmelzprozeß ebenfalls unter Rühren durchführt, - einen Kühlprozeß einleitet - und danach als Suspension dem vorkultiviertem Fermentations-medium zuführt.

Als besonders vorteilhaft beim erfindungsgemäßen Verfahren erweist sich die Präparation und Fermentation im geschlossenen System gemäß Anspruch 2.

Es soll im folgenden das erfindungsgemäße Verfahren näher beschrieben werden.

Das Steroid wird in herkömmlicher Weise bei Temperaturen von 90 °C bis 95 °C in einem wäßrigen Medium, welches mit einem Emulgator, wie beispielsweise Prohalyt G® 20-40 oder Tween® versetzt ist, geschmolzen.
Der Schmelz- und Kühlprozeß wird erfindungsgemäß unter intensivem Rühren durchgeführt, um eine feine Dispersion des Steroids im wäßrigen Medium zu erzielen.
Es hat sich erwiesen, daß mit Dispergierrührwerken, wie Ultraturrax® oder Rotostat® eine sehr feine Steroiddispersion hergestellt werden kann, da diese Rührwerke eine hohe Scherkraft erzeugen.

Erfindungsgemäß wird die feine Schmelzdispersion nicht dem Fermentationsmedium unmittelbar zugeführt, sondern es wird das Steroid innerhalb der Schmelzdispersion durch einen sich dem Schmelzvorgang anschließenden Kühlprozess in feinverteilter Form zum Erstarren gebracht.
Nach einer solchen Erstarrung kann die so erzeugte Steroidsuspension problemlos in das Fermentationsmedium eingebracht werden, ohne daß im Fermenter Verklebungen der Steroidsubstanz zu verzeichnen sind. Um Kontaminationen zu vermeiden werden Präparation und Zugabe in einem geschlossenen System durchgeführt.

Nachweislich ist für eine vollständige Umsetzung des Steroids im Fermentationsmedium ein definiertes Steroid-Kornspektrum von 5µm bis 20 µm besonders günstig, da zu feine Partikel zum Aufrahmen im sich bei der Fermentation bildenden Schaum neigen und dabei im Stoffaustausch behindert sind. Größere Teilchen werden langsamer umgesetzt.
Dieses Kornspektrum kann mit den aufgeführten Rührwerkzeugen mit hoher Scherkraft über die Wahl der Drehzahl einfach und reproduzierbar hergestellt werden

Im erfindungsgemäßen Verfahren erfolgt der mengenmäßige Zusatz des Emulgators in Abhängigkeit vom Gehalt der mikrobiologisch umzusetzenden Steroidsubstanz.
So können erfindungsgemäß Substanzen unterschiedlichen Gehalts, auch Recyclingprodukte, aus dem Prozeß eingesetzt werden.
Es wird für ein 85%-iges Produkt eine Emulgatormenge verwendet, die bezogen auf die Substanzmenge der 4 bis 5fachen Menge entspricht, die für ein 98%-iges Produkt eingesetzt wird.

Als besonders günstig hat sich erwiesen, dem wäßrigen Medium zur Präparation eine Menge von < 1% eines bekannten Antischaummitels zuzusetzen, welches dazu dient, die Schaumbildung während der sehr intensiven Dispergierung des Steroids zu unterdrücken und gleichzeitig in der ersten Fermentationsphase nach der Steroidzugabe eine Schaumbildung zu behindern.

Die Vorteile des erfindungsgemäßen Verfahren besteht darin , daß bei der Herstellung von 13-substituierten 3-Methoxy-8,14- seco-1,3,5(10),9(11)-gonatetraen-14,17-dionen in wäßrigen Fermentationsmedien durch Suspendieren des Steroids in Gegenwart eines Emulgators und eines Antischaummitels es ermöglicht wird, die Steroidverbindunge als wäßrige Suspension in einem definierten Kornspektrum dem Fermentationsmedium zuzusetzen.
Damit verbunden wird eine gezielte nahezu vollständigen Umsetzung des steroids im Fermentationsmedium realisiert.
Mit Hilfe des erfindungsgemäßen Verfahrens gelingt es, die herkömmlichen Verfahren zur Präparation und Fermentation für 3-Methoxy-8,14-seco-1,3,5(10),9(11)-gonatetraen-14,17-dione wesentlich zu verbessern. Die Erfindung soll durch die nachfolgenden Ausführungsbeispiele näher erläutert, jedoch nicht eingeschränkt werden.

### Beispiel 1

In einem Gefäß werden 5 l Wasser vorgelegt und nacheinander 80 ml Prohalyt G 20-40® und 50 ml Polypropylenglycol zugesetzt. Der Gefäßinhalt wird unter Rühren auf 90 °C über den Mantel mit Dampf erhitzt.
Als Rührer wird ein Ultraturrax® verwendet.
Bei einer Drehzahl von 6000 min⁻¹ werden 1,6 kg 3-Methoxy-8,14-seco-1,3,5(10),9(11)-gonatetraen-13-methyl-14,17-dion mit einem Gehalt von
93 % eingerührt und geschmolzen. Dabei wird die Temperatur der Schmelze ½ h zwischen 90 °C und 95 °C gehalten.
Dann wird die Drehzahl auf 8000 min ⁻¹ erhöht und anschließend der Kühlprozess eingeleitet. Es wird auf eine Temperatur von 20 °C bis 25 °C gekühlt und ½ Stunde in diesem Temperaturbereich gerührt.
Die so hergestellte Suspension wird dem Fermentationsmedium zugesetzt. Die Fermentation wird in herkömmlicher Weise durchgeführt und nach erfolgter Umsetzung werden ca. 1,3 kg 3-Methoxy-8,14-seco-1,3,5(10),9(11)-gonatetraen-13-methyl-17ß-ol-14-on über eine extraktive Aufarbeitung isoliert.

### Beispiel 2

51 Wasser werden vorgelegt und nacheinander 40 ml Tween® und 50 ml Polypropylenglycol zugesetzt und entsprechend Beispiel 1 erhitzt sowie gerührt.

Es werden 600g 3-Methoxy-8,14- seco-1,3,5(10),9(11)-gonatetraen-13-methyl-14,17-dion eingerührt und entsprechend Beispiel 1 geschmolzen, gekühlt und dem verwendeten Fermentationsmedium zugeführt.

Nach erfolgter mikrobiologischer Umsetzung und extraktiver Aufarbeitung erhält man ca. 400 g 3-Methoxy-8,14-seco-1,3,5(10),9(11)-gonatetraen-13-methyl-17α-ol-14-on.

### Beispiel 3

Wasser, Emulgator und Tensid werden entsprechend Beispiel 1 erhitzt. Als Rührer wird ein Rotostat® verwendet.
Bei einer Drehzahl von 3000 min⁻¹ werden 2,2 kg 3-Methoxy-8,14-seco-1,3,5(10),9(11)-gonatetraen-13-methyl-14,17-dion mit einem Gehalt von 98% eingerührt und entsprechend Beispiel 1 behandelt.
Anschließend wird die Suspension dem Fermentationsmedium zugesetzt.
Nach erfolgter mikrobiologischer Umsetzung und Isolierung werden ca. 1,8 kg 3-Methoxy-8,14-seco-1,3,5(10),9(11)-gonatetraen-13-methyl-17β-ol-14-on erhalten.

### Beispiel 4

5l Wasser, 70 ml Emulgator und ein Gemisch aus 45 ml Maiskeimöl und 5 ml Polypropylenglycol als Antischaummittel werden wie in Beispiel 1 erhitzt.
Darin werden 1,5 kg 3-Methoxy-8,14-seco-1,3,5(10),9(11)-gonatetraen-13-ethyl-14,17-dion mit einem Gehalt von 97 % bis 99)% eingerührt und geschmolzen.
Es wird weiter wie in Beispiel 1 verfahren.
Nach erfolgter Abkühlung wird die Suspension 2 Stunden bei einer Temperatur von ca. 20 °C gerührt und dann dem Fermentationsmedium zugesetzt.
Nach erfolgter mikrobiologischer Umsetzung und entsprechender Aufarbeitung erhält man ca. 1,1 kg 3-Methoxy-8,14-seco-1,3,5(10),9(11)-gonatetraen-13-ethyl-17β-ol-14-on.

### Beispiel 5

51 Wasser, 120 ml Prohalyt® und 50 ml Polypropylenglycol werden entsprechend Beispiel 1 vorgelegt und 1 erhitzt.

Es werden 500g eines 85%-igen 3-Methoxy-8,14-seco-1,3,5(10),9(11)-gonatetraen-13-ethyl-14,17-dion zugesetzt und entsprechend Beispiel 1 geschmolzen und gekühlt.
Nach erfolgter Abkühlung wird die Suspension in einem Temperaturbereich von 20 °C bis 25 °C 2 Stunden gerührt und dann dem Fermentationsmedium zugeführt.
Nach erfolgter Umsetzung und anschließender Produktisolierung erhält man ca. 340 g 3-Methoxy-8,14-seco-1,3,5(10),9(11)-gonatetraen-13-ethyl-17β-ol-14-on.

## Patentansprüche

1. Verfahren zur Präparation und Fermentation von 13-substituierten 3-Methoxy-8,14-seco-1,3,5(10),9(11)-gonatetraen-14,17-dionen in wäßrigen Fermentationsmedien durch Suspendieren des Steroids im Wasser in Gegenwart von Tensiden,
**dadurch gekennzeichnet, daß** man
- 1 Teil Steroidverbindung 2 bis 9 Teilen eines auf
80 bis 95 °C erhitzten Wasser-Emulgator-Antischaummittels
mit Prohalyt G 20-40®, Tween® als Emulgator und Polypropylenglycol, Polypropylenglycol/Maiskeimöl als Antischaummittel
im Verhältnis 2,5 : 1 bis 1 : 2
und einer Menge von 1 bis 5 % bezogen auf Wasser,
wobei weiterhin die Menge an Emulgator bestimmt wird durch den Gehalt der Steroidverbindung,
in herkömmlicher Weise unter Rühren zusetzt und schmilzt,
wobei man den Schmelzprozeß ebenfalls unter Rühren durchführt,
- einen Kühlprozeß einleitet
- und danach als Suspension dem vorkultiviertem Fermentationsmedium zuführt.

2. Verfahren nach Anspruch 1,
**dadurch gekennzeichnet, daß**
man die Präparation und die Fermentation im geschlossenen System durchführt.

## Claims

1. Process for the preparation and fermentation of 13-substituted 3-methoxy-8,14-secogona-1,3,5(10),-9(11)-tetraene-14,17-diones in aqueous fermentation media by suspending the steroid in water in the presence of surfactants, **characterized in that**
- 1 part of steroid compound is added to 2 to 9 parts of water-emulsifier-anti-foaming agent which is heated to 80 to 95°C and comprises
Prohalyt G 20-40® or Tween® as emulsifier and polypropylene glycol or polypropylene glycol/maizegerm oil as anti-foaming agent in a ratio of 2.5 : 1 to 1 : 2
and in an amount of 1 to 5% based on water,
where, in addition, the amount of emulsifier is determined by the content of the steroid compound,
in a conventional manner with stirring and the steroid compound is melted,
the melting process likewise being carried out with stirring,
- a cooling process is initiated
- and thereafter the steroid compound is fed as suspension to the pre-cultured fermentation medium.

2. Process according to Claim 1, **characterized in that** the preparation and the fermentation are carried out in a closed system.

## Revendications

1. Procédé de préparation et de fermentation de 3-méthoxy-8,14-seco-1,3,5(10),9(11)-gonatétraène-14,17-diones, substitués en position 13, dans des milieux de fermentation aqueux, par suspension du stéroïde dans l'eau en présence d'agents tensioactifs, **caractérisé en ce que** l'on ajoute, en agitant de la manière classique à 1 partie du composé de stéroïdes, de 2 à 9 parties d'un agent antimousse/d'un agent émulsifiant/d'eau, échauffé à une température de 80 à 95°C, avec le produit Prohalyt G20-40®, Tween®, en tant qu'agent émulsifiant, et avec du polypropylèneglycol, du polypropylèneglycol/de l'huile de germe de maïs, en tant qu'agent antimousse, dans le rapport 2,5 : 1 à 1 : 2 et avec une quantité de 1 à 5 % par rapport à l'eau, la quantité d'émulsifiant étant en outre déterminée par la teneur du composé de stéroïdes, et **en ce que** l'on fait fondre le mélange, le procédé de fusion étant également effectué sous agitation,
**en ce que** l'on amorce un procédé de refroidissement,
et que l'on achemine ce mélange par la suite en tant que suspension au milieu de fermentation pré-cultivé.

2. Procédé selon la revendication 1, **caractérisé en ce que** l'on effectue la préparation et la fermentation sous la forme d'un système fermé.
